# EUROPEAN PATENT APPLICATION

(11) **EP 3 254 688 A1**
(43) Date of publication of application: **13.12.2017**
(21) Application number: 15795367.0
(22) Date of filing: 19.05.2015
(51) Int. Cl.: A61K 38/28, A61K 47/30, A61K 47/36, A61K 47/42, A61P 3/10

(54) **INSULIN-CONTAINING PROLONGED-ACTION PREPARATION**

(30) Priority: 22.05.2014 RU 2014120626
(71) Applicant: Sabetckij, Vladimir Andreevich, 192288 St. Petersburg (RU)
(72) Inventor: Sabetckij, Vladimir Andreevich, 192288 St. Petersburg (RU)
(74) Representative: Avidity IP
(86) International application number: PCT/RU2015/000317
(87) International publication number: WO 2015/178806

(57) **Abstract**

The invention relates to the field of biotechnology and medicine, and specifically to insulin-containing injectable formulations used, in particular, for treating diabetes mellitus. The present formulation comprises insulin and a pharmacologically acceptable polymer having a hydrodynamic diameter of more than 4.5+/-0.5 nanometers. As said polymer, at least one polymer having a molecular weight of 20-70 kDa may be optimally used, and is selected from a group including: dextran, polyethylene glycol and albumin. As regards insulin formulations used, it is possible to use human recombinant insulin and genetically engineered analogs thereof used in the commercial formulations. Experiments have shown that using the aforementioned polymers provides insulin formulations with a prolonged effect without the use of chemical or genetic methods of modifying insulin molecules. The method of long acting formulations preparing is not dependent on biotherapeutics and thus it can be used over range of therapeutic proteins.

## Description

### TECHNICAL FIELD

The invention relates to the field of biotechnology and medicine, namely to the insulin-containing injectable formulations used in particular for the treatment of diabetes.

### BACKGROUND

Currently, medicines for the treatment of diabetes mellitus (commonly also called "diabetes") are the most important pharmaceutical products in the world market. In 2012, sales of only insulin preparations was 20.8 billion dollars, and it is assumed that it will reach 32.5 billion in 2018. Worldwide, in 2013, 382 million people suffered from diabetes and, in according to many forecasts, by 2030 their number will increase to 592 million.

Diabetes is a metabolic disorder caused by absolute or relative insulin deficiency, which is the only hypoglycemic hormone, and the main symptom of diabetes mellitus is hyperglycemia. Therefore, losses of the synthesizing insulin beta cells in the pancreas of first type diabetes mellitus patient or insulin resistance and slow loss of beta cells in patients with second type diabetes. Diabetes is also associated with a number of chronical complications, including microvascular diseases such as retinopathy, nephropathy and neuropathy, and macrovascular diseases, such as heart attacks and strokes. [RU2358738, 2009].

For the treatment of diabetes, other than insulin hypoglycemic agents can be used for example such as insulin secretagogues, drugs sensitizing tissues to insulin action (metformin), as well as inhibitors of α-glucosidase to prevent splitting of sugars in the intestine [RU2358738, 2009].

Although the possibility of application of these hypoglycemic agents has been demonstrated by clinical practice, their practical use is associated with a number of problems. Because diabetic patients have the significantly reduced ability to secrete insulin, effective stimulators of the secretion of insulin gradually decrease and eventually stop secretion completely and the main drug used to treat diabetes remains insulin. (V. Sabetsky, J. Ekblom "Insulin: A new era for an old hormone", Pharmacological Research 61 (2010), 1-4).

Insulin was first isolated from the pancreas of dogs in Canada in 1921 by Banting and Best in laboratory of McLeod. Studies have shown that human insulin molecule consists of two chains of amino acids; A-chain comprises 21 amino acids and B-chain - 30. These chains are interconnected by two disulfide bridges, a third disulfide bridge connects two remote amino acid of the A-chain. In aqueous solutions, insulin molecule has globular structure necessary for the manifestation of its biological activity. Methods for producing insulin were originally based on extracts from pancreas of cattle and pigs. However, their amino acid compositions differ from human insulin, bovine insulin at two positions and porcine insulin at one position, leading to the appearance in patient adverse side effects, such as allergy. Thanks to advances in genetic engineering in the 70s the technology of obtaining protein drugs has been developed by creating a recombinant DNA and insulin was produced mainly by bacteria or yeast cells. In the 80s, the production of recombinant human insulin of high quality began and the main problem in the development of drugs based on it was the creation of products with optimal pharmacokinetics (PK).

As a rule, the treatment of patients with diabetes includes the use of a combination of insulin with fast (short) and long (sustained) effect. The short-acting insulin should quickly reach a peak of activity in accordance with the rise in blood glucose associated with meal, and terminated upon its fall and long-acting insulin, on the contrary, should over time provide a certain blood glucose level between meals.

Currently commercially available "fast" insulins are "Lispro" in which the amino acid sequence of the B chain residues inverted proline and lysine-B28-B29, "Glulysin" (LysB3 , GluB29 human insulin) and «Aspart» (AspB28 human insulin), in its molecule the residue of proline at position B28 of the B chain is replaced by an aspartic acid residue. Such modification of the human insulin molecule have the reduced propensity of human insulin molecules to aggregate and facilitate the absorption of the hormone at the injection site [Setter SM, Corbett CF, Campbell RK, White JR Ann. Pharmacother., 2000; v.34, p. 1423-1431]. This has led to a significant reduction in the start time of action of drugs increase the maximum achievable concentration of drugs in blood and more rapid recovery to baseline hormone levels. [Simpson KL, Spenser CM. Drugs, 1999, v.57, r.759-765].

It has been shown that prolonged action exhibit insulin analogs in which at least one amino acid at position B1 - B6 is replaced by lysine or arginine [WO 92/00321, ER0368187]. The most efficient of this group of drugs is glargine (Sanofi-Aventis), which allowed the substitution of amino acids to shift the protein isoelectric point towards neutral pH. The injection solution of glargine having pH 4, when administered under the skin, slowly forms a micro-precipitate, which slowly dissolves and provides flat profile of "time - concentration" curve within 24 hours. Glargine appeared in 2000 and dominates the market of long-acting insulin over the past 10 years.

The company Novo-Nordisk, developed insulin degludec (Tresiba U100, Tresiba U200), which is an insulin analogue modified with a C16 fatty acid via a linker. Insulin degludec, after subcutaneous administration, forms soluble multimers that slowly dissolve and this leads to ultra-prolonged action. The formulation is registered in Europe and Japan, but the FDA (Food and Drug Administration, US) refused the Danish pharmaceutical company Novo Nordisk registration antidiabetic drugs Tresiba (insulin degludec) and Ryzodeg (insulin degludec / insulin aspart) in the United States, arguing it is unfavorable safety profile. [FDA Rejects Novo Nordisk's Insulin Degludec [http://www.medscape.com/viewarticle / 779,077]. Eli Lilly currently produces two long acting insulin formulations based on insulin lispro modified by PEGylation or in the form de protamine suspension [Caparrotta TM, Evans M., «PEGylated insulin Lispro, (LY2605541) -a new basal insulin analogues Diabetes ObesMetab. 2014 May; 16 (5): 388-95); Diabetes Dario Giugliano, Katherine Esposito «Efficacy and Safety of Insulin Lispro Protamine Suspension as Basal Supplementation in Patients With Type 2», Advances in Endocrinology and Metabolism. 2012; 3 (3): 99-108].

The disadvantage of genetically engineered insulin analogues is their only partial compliance with the structure of human insulin.

Ongoing studies aimed at achieving a prolongation of action of human insulin may be divided into two main directions: to obtain prolongation effect due to chemical and/or genetic engineering techniques, leading to modifications of the insulin molecule and the use of physical and chemical methods without changing the insulin molecule.

The use of chemical modification of insulin (PEGylation and acylation with fatty acids) for the prolongation of the action most clearly manifested in the development of technology TransCon, allowing the sustained release of insulin from the subcutaneous depot.

In 2007, Sanofi-Aventis filed a patent application [US20120183616, 2013] on the technology of chemical modification of insulin molecule by the addition of PEGylated insulin to biodegradable hydrogel by a linker (TransCon Linker and Hydrogel Insulin). Sustained release of insulin from the subcutaneous depot for two weeks has been demonstrated in the experiments on rats. However, it should be noted that the inevitable use of large doses of insulin and an extended stay in the place of injected insulin, especially in the form of suspensions, is fraught with the possibility of absorption of large particles suspensions by macrophages, which can lead to complications and impossibility of selection of an optimal dosing regimen. In addition, the increase in the duration of a term of more than one day lead to the need for training of patients for titration and strict compliance regime of administration.

Physico-chemical methods of prolonging the insulin action use the fact that at physiological pH the insulin molecule is negatively charged. So, promising is an obtaining of insulin complexes with organic polycations and divalent metal cations to slow the formation of insulin monomers having very fast absorption. The group is represented in the market by Protafan, NPH, Lente, Semilente, Insulong, Aktrafan. Unlike glargine, which is a clear solution, these agents are suspensions and their accurate dosing problem arising.

The major drawback of these drugs is the impossibility to obtain the flat (peakless) pharmacokinetic profile of the curve, which leads to an increase in hypoglycemia events at higher doses. The duration of action of this group is proportional to their insulin dose. The peak effect occurs in about 6-8 hours.

The closest to the claimed invention is one developed earlier by the author (US7544656, 2009) where the long-acting insulin formulation is a mixture of insulin and highly porous biocompatible and biodegradable microspheres. The suspension of microspheres in an aqueous solution of insulin being injected with a syringe subcutaneously or intramuscularly to experimental animals (rabbits and mice) form depot. Release of insulin from the depot is slowed down considerably since the molecules of insulin had to move through the highly porous structure of the microspheres. The strong disadvantage of the resulting formulation were relatively high viscosity of the suspension and, as a consequence, the use of large diameter needle for injection, and the very slow (months) biodegradation of the microspheres.

Problems to be solved by the author, was to develop long acting insulin formulations with use of special components, which provide slow sustained release from depot of both natural insulins, insulin analogues, and their biosimilars, including their physically and/or chemically modified versions.

### SUMMARY OF THE INVENTION

The author explored the possibility to use the effect, discovered in 1896 by Starling. In experiments on dogs, Starling showed that the injection of isotonic physiological solution (normal saline) leads to dilution of blood in the leg vein, while the introduction of serum instead of the saline did not provide the dilution effect. Starling explained the results by the fact that the serum contains components that retain water and have a size that prevent their absorption into the blood through the capillaries [Starling E.H. On the absorption of fluids from the connective tissue spaces. J Physiol 1896; 19: 312-326]. In addition, colloidal osmotic pressure (COP) of present in the plasma high molecular weight proteins (predominantly albumins) influenced on the water retention in the blood. The author suggested that the administration of injectable formulations containing biocompatible polymers in concentrations providing the COP balance between the plasma and the interstitial fluid at the site of injection can significantly slow the output of insulin molecules from the subcutaneous depot and delay their absorption into the circulation.

Experiments made have demonstrated that insulin solutions combined with pharmacologically acceptable polymers with a hydrodynamic diameter greater than 5+/-0.5 nanometers that prevents its absorption into the blood through the walls of capillaries in the tissues provide strong prolong effect following subcutaneous administration in animals and humans. Polymers of choice used are biocompatible water-soluble polymers with a molecular weight of 20-100 kDa, for instance, dextrans, polyethylene glycols, polyvinyl-pyrrolidones, and albumins.

The polymers of choice are ones that are really the ones used for the production of plasma-colloidal solutions (CRC) and in the preparation of modified proteins including insulins [ADOCIA, US20120094902; Eli Lilly LY2605541].

### Industrial Applicability

As a component of the inventive formulation, the human recombinant insulin, and its genetically engineered counterparts can be used. Sustained-release formulations can be made by mixing the ingredients or solutions or suspensions containing insulins. The optimum ratio of the ingredients are chosen experimentally starting from the particular use of insulin and the polymer, as a rule, the polymer content of 5-10% by weight of the total weight of formulations.

Formulations claimed do not contain potentially hazardous components and their manufacturing does not require the use of sophisticated equipment what significantly reduces the cost and time required to bring new drugs to the market and their introduction into clinical practice.

The essence and advantages of the claimed invention are demonstrated by the following examples.

### Example 1

0.6 g of dextran 70 kDa (Pharmacosmos, Denmark) was dissolved in 9.4 g of insulin Astrapid formulation (100 IU/ml, Novo Nordisk), filtered through a sterilizing membrane of 0.22 microns and dispensed into sterile 2 ml eppendorfs. The dynamic viscosity of the solution was of 5.0 mPa-s, colloid osmotic pressure (COP) of 58 mm Hg. The 6% solution made was marked as AD70.

1.0 g of dextran 40 kDa (Pharmacosmos, Denmark) was dissolved in 9.0 g of insulin Astrapid (100 IU/ml, Novo Nordisk), filtered through a sterilizing membrane of 0.22 microns and dispensed into sterile 2 ml eppendorfs. The dynamic viscosity of the solution was of 7.0 mPa-s, colloid osmotic pressure (COP) of 90 mm Hg. The 10% solution made was marked as AD40.

0.8 g of poly(ethylene glycol) (PEG) 20 kDa (BioUltra, 20,000, Sigma-Aldrich) was dissolved in 9.2 g of insulin Astrapid (100 IU/ml, Novo Nordisk), filtered through a sterilizing membrane of 0.22 microns and dispensed into sterile 2 mL eppendorfs. The dynamic viscosity of the solution was of 11 mPa-s, colloid osmotic pressure (COP) of 40 mm Hg. The 8% solution made was marked as AP20.

Four Chinchilla rabbits (weight 3.5 +/- 0.2 kg, males) were marked as R1, R2, R3, and R4 and involved in the conducted experiments. The rabbits were injected subcutaneously with 0.05 ml (4.6 +/- 0.1 IU) of the solutions above, R1 - has got insulin Actrapid HM (control), R2 - AD40, R3 - AD70, R4 - AP20. After 15 minutes and 180 minutes after administration in rabbits, 1 ml of blood was taken from the ear vein for determination of human insulin by enzyme-linked immunosorbent assay (ELISA). To ovoid the strong hypoglycemia, blood glucose level of the rabbits was measured with portable glucometer «ContourTS» (Bayer) every 15 minutes. If the glucose concentration reached 2.0 mml/l, the animal was administered intravenously 10 ml of 20% glucose solution and withdrawn from the experiment. Experimental data obtained are shown in Table 1.

**Table 1**

| | | Insulin concentration at 15min, (µIU/ml) | Insulin concentration at 180 min, (µIU/ml) |
|---|---|---|---|
| R1 | Actrapid HM | 117.0 | 36.8 |
| R2 | AD40 | 59.0 | 44.1 |
| R3 | AD70 | 50.9 | 42.1 |
| R4 | AP20 | 47.8 | 39.3 |

The results obtained may be interpreted as demonstrating significant delay effect of insulin absorption from the site of subcutaneous administration for all experimental samples compared with the control. Mechanism of the effect can be explained by forming depot with COP, which is equal to one of plasma, and change in the equilibrium concentrations of the insulin monomers, dimers, and hexamers.

### Example 2

1.0 g of dextran (dextran 40 kDa, Pharmacosmos, Denmark) was dissolved in 9.0 g of the prolonged action insulin Humulin NPH (100 IU/ml, Eli Lilly) and dispensed into sterile 2 ml eppendorfs (because the insulin formulation is a slurry, filtering it through a 0.22 micron membrane was not used). The dynamic viscosity was of 7.0 mPa-s, colloid osmotic pressure of 90 mm Hg. The prepared 10% solution was marked as HD40.

The experiment involved six Chinchilla rabbits (weight 3.5 +/- 0.2 kg, males), designated as R1, R2, R3, R4, K5, R6.

Rabbits R1, R2, R3 were subcutaneously injected with 50 µl (4.6 +/- 0.1 IU) of Humulin NPH (control) and rabbits R4, R5, R6 subcutaneously injected with 50 µl (4.6 +/- 0.1 IU) of the HD40. 60 and 1440 minutes after administration in rabbits, 1.0 ml of blood was taken from the ear vein for the determination of human insulin by enzyme-linked immunosorbent assay (ELISA).

After 60 minutes in the blood of rabbits R1, R2, R3 human insulin content was 53 +/- 7 µIU/ml and of R4, R5, and R6 31 +/- 11 µIU/ml. After 1440 minutes (one day), the blood of R1, R2, R3 did not contain human insulin at all, while in the blood of rabbits R4, R5, R6 insulin content was at the level of 14 +/- 5 µIU/ml.

The results obtained demonstrate that the HD40 has a much longer effect than Humulin NPH (control), which action is 14-16 hours.

### Example 3

0.5 g of dextran (dextran 70 kDa, Pharmacosmos, Denmark) was dissolved in 9.5 g of insulin NovoRapid (100 IU / ml, Novo Nordisk), filtered through a sterilizing membrane of 0.22 microns and dispensed into sterile 2 ml eppendorfs. The dynamic viscosity of the solution is 5.0 mPa-s, colloid osmotic pressure of 58 mm Hg. The prepared solution was marked as ND70.

1.0 g of dextran (dextran 40 kDa, Pharmacosmos, Denmark) was dissolved in 9.0 g of insulin NovoRapid, filtered through a sterilizing membrane of 0.22 microns and dispensed into sterile 2 ml eppendorfs. The dynamic viscosity of the solution was 7.0 mPa-s, colloid osmotic pressure of 90 mm Hg. The prepared solution was marked as ND40.

1.0 g of PEG 20 kDa (BioUltra, 20,000, Sigma-Aldrich) was dissolved in 9.0 g of insulin NovoRapid, filtered through a sterilizing membrane of 0.22 microns and dispensed into sterile 2 ml eppendorfs. The dynamic viscosity of the solution was of 10 mPa-s, colloid osmotic pressure of 40 mm Hg. The prepared solution was marked as NR20.

The experiment involved four Chinchilla rabbits (weight 3.5 kg, males) marked as R5, R6, R7, and R8. The rabbits were injected subcutaneously with 50 µl (4.6 +/- 0.1 IU), R5 with insulin NovoRapid (control), R6 with ND40, R7 with ND70, and R8 with NP20.15 minutes and 180 minutes after administration in rabbits, 1 ml of blood was taken from the ear vein for the determination of human insulin concentration by ELISA.

The blood glucose content was determined using a portable glucometer «ContourTS» (Bayer). If the content was below 2.0 mmol/l, the animal was to be administered intravenously with 10 ml of 20% glucose solution and withdrawn from the experiment.

The experimental data are shown in Table 2

**Table 2**

| | ΠpeπapaT | Insulin concentration at 15 min, (µIU/ml) | Insulin concentration at 180 min, (µIU/ml) |
|---|---|---|---|
| R5 | NovoRapid | 149.0 | 7.7 |
| R6 | ND40 | 63.1 | 27.9 |
| R7 | ND70 | 61.0 | 31.7 |
| R8 | NP20 | 54.9 | 31.3 |

The results obtained can be interpreted as confirming strong prolonged effect. Insulin Aspart is rapid-acting insulin analogue obtained by recombinant technology, wherein the amino acid proline at position B28 is replaced by aspartic acid. The purpose of such replacement is the more rapid dissociation of stabilized by zinc hexamers to dimers and monomers of insulin analog.

For further experiments on human, dextran 40kDa and dextran 70 kDa were chosen. This choice was made to determine the impact of MW and the COP on the protraction effect in experiments on human.

### Example 4

Pure insulin substance was isolated from commercial insulin Actrapid HM with preparative HPLC and dissolved in PBS buffer containing (pH 7.2 - 7.4) to reach100 IU/ml solution without zinc (Zn0). 0.6 g of dextran 70 kDa was dissolved in the obtained solution and marked as D70Zn0, and 1g of dextran 40 kDa was dissolved in 9g of the solution and marked as D40Zn0.

0.1 ml (about 10 IU) of the solutions above were injected subcutaneously (femoral area) to healthy volunteer (BMI 25, needle 28.5 G). Control of blood glucose was carried out every 15 minutes with a portable glucometer Contour TS (Bayer).

Baseline blood glucose (after 12 hours without food intake) was 4.9 mmol/ l in the experiment with the D40Zn0 and 5.0 mmol / l with D70Zn0. Both formulations started about 60 minutes after administration. The D40Zn0 was maintaining the blood glucose level of 3.8 +/- 0.5 mmol / l for 10 hours, the D70Zn0 4.0 +/- 0.6 mmol / l for 8 hours. It should be noted that pharmacodynamics (PD) profile for both formulations was peakless (flat).

### Example 5

Under conditions of the Example 4, dose 15 IU (0.15 ml) of D40Zn0 have been used to evaluate the dosage influence on the magnitude and duration of the prolonged effect. Parallel pharmacodynamics, evaluation of pharmacokinetics was studied by insulin and C-peptide was measuring in the clinical laboratory.

The effect started within 60 minutes. Glucose was maintained at 3.6 +/- 0.3 mmol/l for 12 hours with no peaks. Pharmacokinetics data are shown in Table 3.

**Table 3**

| Time (min) | 0 | 60 | 135 | 240 | 360 | 480 | 540 | 600 |
|---|---|---|---|---|---|---|---|---|
| Insulin (µIU/ml) | 4.8 | 16.1 | 16.0 | 15.6 | 11.9 | 8.0 | 7.6 | 5.6 |
| C-peptide (ng/ml) | 1.23 | 0.71 | 0.29 | 0.12 | 0.07 | 0.05 | 0.14 | 0.11 |

Data on the pharmacokinetics demonstrate the absence of peak insulin that is in full compliance with the data on pharmacodynamics. It is noteworthy that increasing the dose did not result in a significant reduction in glucose level (no hypoglycemia) and the protraction effect was increased significantly compared to the dose of 10 IU.

### Example 6

AD40 formulation (see Example 1) differs from Actrapid HM only by presence of clinical dextran 40 kDa, which does not react with the components of the commercial formulation. The aim of the experiment was to compare the pharmacokinetics and pharmacodynamics of the AD40 in human with the results of the previous two examples. We used two doses of the AD40 - 10 and 15 IU.

A dose of 10 IU AD40 formulation showed onset of action within 60 minutes after the administration and maintenance of glucose at level of 3.3 +/- 0.3 mmol / l for 14 hours. Pharmacokinetics data are presented in Table 4.

**Table 4**

| Time (min) | 0 | 60 | 135 | 240 | 360 | 540 |
|---|---|---|---|---|---|---|
| Insulin (µIU/ml) | 4.8 | 13.0 | 13.5 | 11.4 | 10.4 | 7.6 |
| C-peptide (ng/ml) | 1.54 | 0.18 | 0.28 | 0.14 | 0.11 | 0.09 |

Dose 15 U launched after 60 minutes and for 22 hours, blood glucose was maintained at 3.7 +/- 0.3 mmol/l and then for 3 hours returned to baseline. Pharmacokinetics data are presented in Table 5.

**Table 5**

| Time (min) | 0 | 60 | 120 | 240 | 480 | 720 |
|---|---|---|---|---|---|---|
| Insulin (µIU/ml) | 6.2 | 13.9 | 13.5 | 13.4 | 13.6 | 9.9 |
| C-peptide (ng/ml) | 1.32 | 1.13 | 0.70 | 0.49 | 0.10 | 0.05 |

According to manufacturer (Novo Nordisk), the used in the study experiments Actrapid HM is short-acting insulin, produced by recombinant DNA technology. It starts in 30 min, the maximum concentration (Cmax) of insulin in plasma is reached within 1.5-2.5 hours, and maximum action is achieved between 2.5-5 hours after administration. The Actrapid's action lasts during 7-8 hours.

Obviously, the pharmacokinetics and pharmacodynamics of the AD40 differ significantly from those of insulin Actrapid HM. After addition of 40 kDa dextran into Actrapid, solution became possessing of colloidal osmotic pressure (COP) of 90 mm Hg, remaining isotonic for the low molecular weight components. This led to a prolongation of action in half for the dose of 10 IU, and three times for the dose of 15 U AD40 drug and the complete absence of the peak concentrations of insulin and glucose.

As shown in the conducted experiments, the use of the above polymers ensures that prolonged effect of insulin formulations without chemical or genetic engineering methods of modification of human insulin molecules. Since the method is not specific, it may be used for a wide variety of therapeutic proteins.

## Claims

1. A long acting insulin formulation, which comprises insulin and pharmaceutical grade, water-soluble polymer that provides a long lasting effect and has a hydrodynamic diameter greater than 4.5 +/- 0.5 nanometers.

2. The long acting insulin formulation according to claim 1, **characterized in that** the pharmaceutical grade, water-soluble polymer providing a long lasting effect has a molecular weight of 20-70 kDa and is dextran, or polyethylene glycol, or albumin.

3. The long acting insulin formulation according to claim 1, **characterized in that** the providing long lasting effect polymer concentration is 5-10% by weight.

4. The long acting insulin formulation according to claim 1, **characterized in that** the insulin is recombinant human insulin.

5. The long acting insulin formulation according to claim 1, **characterized in that** the insulin is a recombinant human insulin analog.

6. The long acting insulin formulation according to claim 1, **characterized in that** it comprises a complex of a recombinant human insulin, zinc, and protamine.
